# EUROPEAN PATENT APPLICATION

(11) **EP 4 218 623 A1**
(43) Date of publication of application: **02.08.2023**
(21) Application number: 21872453.2
(22) Date of filing: 22.09.2021
(51) Int. Cl.: A61B 17/17, A61B 17/88, A61F 2/44, A61F 2/46

(54) **END-PLATE PERFORATION DEVICE**

(30) Priority: 23.09.2020 JP 2020159032
(71) Applicant: National University Corporation Tokai National Higher Education and Research System, Nagoya-shi, Aichi 464-8601 (JP)
(72) Inventor: NOZAWA, Satoshi, Gifu-shi, Gifu 501-1193 (JP)
(74) Representative: Petraz, Gilberto Luigi
(86) International application number: PCT/JP2021/034664
(87) International publication number: WO 2022/065324

(57) **Abstract**

The endplate perforator (10) includes a pipe, an operation portion disposed at a rear end of the pipe, a case (41; 141) that is disposed at a front end of the pipe and includes an insertion surface configured to be placed parallel to an endplate (S) of a vertebra (V), a needle member (60; 160) housed in the case (41; 141) and configured to perforate the endplate (S) of the vertebra (V), and a mechanism portion (42; 150) configured move the needle member (60; 160) in a direction perpendicular to the insertion surface in response to an operation of the operation portion to cause the needle member (60; 160) to project or retract relative to the case (41; 141).

## Description

### TECHNICAL FIELD

The present disclosure relates to an endplate perforator.

### BACKGROUND ART

An interbody fusion is a common surgical approach to address spinal injuries and lesions. The interbody fusion is performed for nerve decompression and rest when the spine, such as the lumbar spine, is unstable.

Representative interbody fusions include an anterior interbody fusion, which accesses the spine from the patient's front portion (anterior), and a posterior interbody fusion, which accesses the vertebrae through an incision in the patient's back (posterior).

The anterior interbody fusion is the representative technique in the art and requires a relatively large incision in the abdominal space, but allows for the implantation of largest cages. The posterior interbody fusion is a fusion procedure that does not require a large back incision and allows for access to the spine of the patient in the prone position on an operating table. Thus, the posterior interbody fusion does not require repositioning of the patient on the operating table after the procedure.

In conventional interbody fusions, the intervertebral tissue (intervertebral disk) between vertebral bodies is dissected with a punch, followed by cage insertion and/or bone grafting. Specifically, a cage is inserted into the space between the vertebral bodies formed by dissecting the intervertebral tissue, or a portion of a vertebra cut in the surgery or bones harvested from the pelvis are grafted in the space. This eventually promotes bone fusion and thus fuses the vertebrae.

It is difficult to completely remove the intervertebral tissue during dissection. This is because, if the endplates of the vertebrae are excessively cut in the removal of intervertebral tissue, the cage may sink and become unstable. However, if the intervertebral tissue remains, the bone fusion between the vertebral bodies and the bone graft between the vertebral bodies will be extremely slow, often requiring half a year to one year or longer. The bone fusion rate of interbody fusions is 42% at 6 months, 79% at 1 year, and 87% at 2 years (Kang T. et al. Arch Osteoporosis (2019) 14:74).

On the other hand, breaking the endplates of the vertebrae to allow bone marrow fluid to leak during cage insertion and/or bone grafting is considered to facilitate bone fusion because of the bone marrow stem cells introduced into the space between the vertebral bodies.

The applicant has performed a patent search on interbody fusions and found Patent Literatures 1 to 3.

In Patent Literature 1, a perforator is configured to insert a cutting drill while rotating it from the posterior side of adjacent vertebrae to cut the endplates of both vertebrae sandwiching the intervertebral disk.

In Patent Literature 2, a pair of forceps having a socket member attached to its distal end portion is inserted into a space formed by dissecting the intervertebral tissue (intervertebral disk). The forceps are operated to perforate the endplate of the vertebra with a sharp section of the socket member.

In Patent Literature 3, the endplate of the upper vertebra is obliquely perforated by a bone screw oriented obliquely upward, and the endplate of the lower vertebra is obliquely perforated by a bone screw oriented obliquely downward.

### CITATION LIST

### Patent Literature

Patent Literature 1: Japanese Laid-Open Patent Publication No. 7-8514
Patent Literature 2: United States Patent No. 3875595
Patent Literature 3: Japanese National Phase Laid-Open Patent Publication No. 2018-532492

### SUMMARY OF INVENTION

### Technical Problem

In Patent Literature 1, the entire endplate is destroyed by rotating the cutting drill along the plane of the vertebra endplate. As a result, the inserted cage may sink into the vertebra and be unstable. Additionally, the space between the vertebrae may become narrower, and the screws may become loose failing to maintain the distance between the vertebrae or losing the fixation force. This may protract bone fusion.

Patent Literature 2 aims to fixate vertebrae by placing a bag between the vertebrae. As such, bone fusion will not occur. That is, socket members are driven into the endplates of the vertebrae, and portions of the bag are inserted into the socket members to position the bag. As such, bone fusion is not expected to take place.

In Patent Literature 3, the endplates of upper and lower vertebrae need to be perforated by obliquely placing bone screws in the endplates. The bone screws extending near nerves may damage the nerves, making the surgical procedure difficult.

An objective of the present disclosure is to provide an endplate perforator that forms a bone tunnel that is substantially perpendicular to the endplate regardless of the direction of access to the patient's spine to suitably and easily facilitate bone fusion between the vertebrae.

### Solution to Problem

In one aspect of the present disclosure, an endplate perforator is provided. The endplate perforator includes: a pipe, an operation portion disposed at a rear end of the pipe; a case disposed at a front end of the pipe, the case including an insertion surface configured to be placed parallel to an endplate of a vertebra; a needle member housed in the case and configured to perforate the endplate of the vertebra; and a mechanism portion configured to move the needle member in a direction perpendicular to the insertion surface in response to an operation of the operation portion to cause the needle member to project or retract relative to the case.

With the above configuration, the force by which the needle member is project or retract is transmitted to the endplate of the vertebra without being lost, thereby perforating the endplate of the vertebra in a reliable manner to form a bone tunnel. This allows bone marrow fluid to flow out through the perforated bone tunnel, facilitating bone fusion.

The needle member perforates the vertebra endplate to form a bone tunnel but does not destroy the entire vertebra endplate. As such, the cage inserted after the formation of the bone tunnel will not sink into the vertebra.

Furthermore, the needle member projects and retracts with respect to the case in directions substantially perpendicular to the endplate of the vertebra. Thus, the needle member reliably forms a bone tunnel in the vertebra endplate without damaging nerves extending in the direction in which the vertebrae are aligned.

The length of the projecting needle member is preferably long enough to penetrate through the endplate of the vertebra. Although it depends on the thickness of the endplate of the patient's vertebra, the length is in a range of 1 mm to 15 mm, preferably in a range of 3 mm to 10 mm, more preferably in a range of 4 mm to 7 mm.

Also, the thickness of the needle member is preferably dimensioned not to destroy the vertebra endplate while ensuring the strength of the needle member body. Although it depends on the strength of the endplate of the patient's vertebra, the thickness is in a range of 0.5 mm to 10 mm, preferably in a range of 1 mm to 5 mm, more preferably in a range of 1 mm to 2 mm.

The number and layout of needle members may be adjusted according to the thickness, the area, and the like of the patient's vertebral endplates. For example, for a patient with thin endplates, a fewer number of needle members and/or a longer distance between the needle members may be used. For a patient with thick endplates, a greater number of needle members and/or a shorter distance between the needle members may be used.

### BRIEF DESCRIPTION OF DRAWINGS

Fig. 1A is a plan view of an endplate perforator according to a first embodiment.
Fig. 1B is a side view of the endplate perforator of Fig. 1A.
Fig. 2 is an enlarged cross-sectional view of the vicinity of an operation member of the endplate perforator of Fig. 1A.
Fig. 3 is a perspective view of the vicinity of a case of the endplate perforator of Fig. 1A.
Fig. 4 is a side view of an actuation portion case in a state in which needle members of the endplate perforator of Fig. 1A are at a retracted position.
Fig. 5 is a cross-sectional side view of the actuation portion case in a state in which the needle members of the endplate perforator of Fig. 1A are at the retracted position.
Fig. 6 is a side view of the actuation portion case in a state in which the needle members of the endplate perforator of Fig. 1A are at a projecting position.
Fig. 7 is a cross-sectional side view of the actuation portion case in a state in which the needle members of the endplate perforator of Fig. 1A are at the projecting position.
Fig. 8 is an explanatory plan view showing a state in which the endplate perforator of Fig. 1A is inserted between vertebral bodies.
Fig. 9 is an explanatory side view of a state in which the endplate perforator of Fig. 1Ais inserted between vertebral bodies.
Fig. 10 is an explanatory side view of a state in which the endplate perforator shown in Fig. 1A is inserted between the vertebral bodies and the needle members project upward.
Fig. 11 is an explanatory side view of a state in which the endplate perforator shown in Fig. 1A is inserted between the vertebral bodies, turned upside down from the state shown in Fig. 10, and the needle members project downward.
Fig. 12 is an explanatory diagram of a state in which a cage is inserted between the vertebral bodies.
Fig. 13 is a cross-sectional side view of an endplate perforator according to a second embodiment in a state in which needle members of the endplate perforator are at a retracted position.
Fig. 14 is a cross-sectional side view of an actuation portion case in a state in which the needle members of the endplate perforator of Fig. 13 are at a projecting position.
Fig. 15 is a transverse cross-sectional view of the essential part showing the relationship between a spline shaft housing hole of a needle member and a spline shaft of the endplate perforator of Fig. 13.
Fig. 16 is a side view of an actuation portion case in a state in which needle members of an endplate perforator according to a third embodiment are at a retracted position.
Fig. 17 is a side view of the actuation portion case in a state in which the needle members of the endplate perforator of Fig. 16 are at a projecting position.
Fig. 18 is an intraoperative image of a use case in which the upper vertebral body is perforated by an endplate perforator.
Fig. 19 is an image of bone fusion between a vertebral body and a part of bone in the intervertebral space that is identified three months after the surgery of Fig. 18.

### DESCRIPTION OF EMBODIMENTS

### First Embodiment

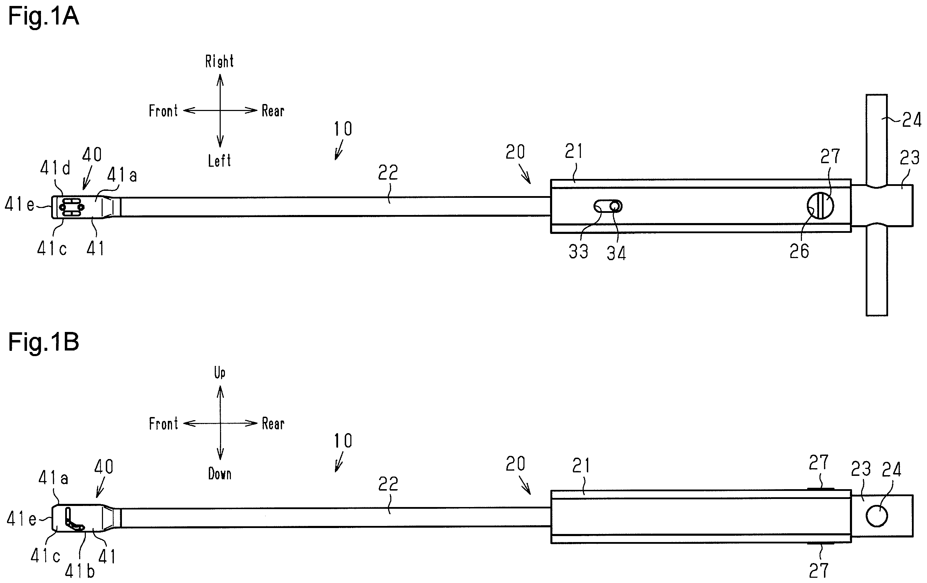

Referring to Figs 1 to 12, an endplate perforator 10 of a first embodiment is now described.

As shown in Figs. 1A and 1B, the endplate perforator 10 includes a shaft portion 20 and an actuation portion 40 provided at the distal end of the shaft portion 20. In the descriptions herein, the distal end side at which the actuation portion 40 is disposed is referred to as a front side, and the opposite side is referred as a rear side. Also, the directions indicated by terms such as "upper", "lower", "left", and "right" are defined with reference to the endplate perforator 10 oriented such that needle members 60 described below project upward.

### Shaft Portion 20

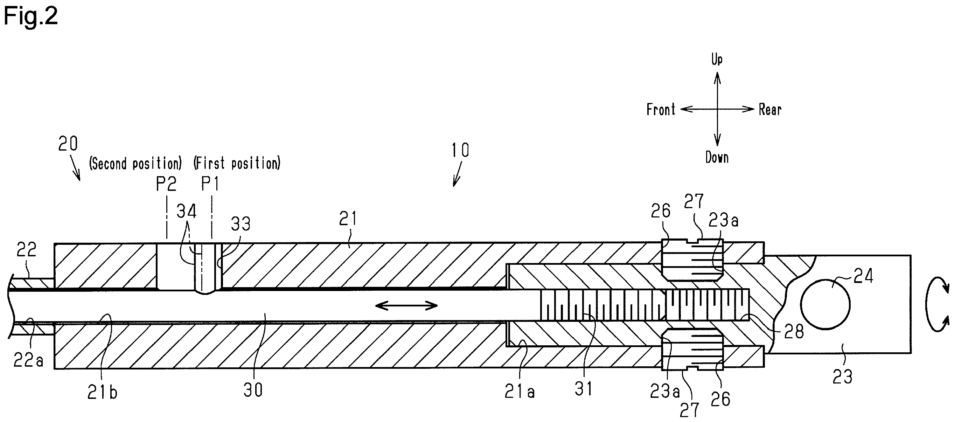

As shown in Figs. 1A, 1B, and 2, the shaft portion 20 includes a grip pipe 21 with a large diameter and an extension pipe 22 with a small diameter. The extension pipe 22 is coaxial with the grip pipe 21 and integrally coupled to the front end of the grip pipe 21. The grip pipe 21 includes a pipe hole 21a with a large diameter on the rear side and a pipe hole 21b with a small diameter on the front side. The pipe holes 21a and 21b are coaxial with each other. The pipe hole 21a opens at the rear end surface of the grip pipe 21, and the pipe hole 21b communicates with the pipe hole 22a of the extension pipe 22.

The total length of the grip pipe 21 and the extension pipe 22 is set to a length that is long enough to perform a posterior interbody fusion. The grip pipe 21 and the extension pipe 22 serve as a pipe.

As shown in Fig. 2, a columnar operation member 23 is inserted in the pipe hole 21a of the grip pipe 21 to be rotatable about the axis of the grip pipe 21. The rear end portion of the operation member 23 projects outward from the opening of the pipe hole 21a. An operation handle 24 is fixed to and extends through the rear end portion of the operation member 23 in a radial direction of the operation member 23. Thus, as shown in Fig. 1A, the operation member 23 and the operation handle 24 form a cross shape in plan view. The operation member 23 and the operation handle 24 serve as an operation portion.

As shown in Fig. 2, the operation member 23 includes a circumferential groove 23a. The circumferential groove 23a is provided in a section of the outer circumferential surface of the operation member 23 that is located within the pipe hole 21a. The circumferential groove 23a is annular and extends over the entire circumference of the operation member 23. Two threaded holes 26 extend through the rear end portion of the grip pipe 21 in the radial direction. A setscrew 27 is threaded in each threaded hole 26. The distal end of the setscrew 27 is inserted in the circumferential groove 23a of the operation member 23. This stops the operation member 23 from moving in the axial directions of the grip pipe 21. The number of threaded holes 26 is not limited to two, and may be one or more.

As shown in Fig. 2, the operation member 23 includes a threaded hole 28 opening at its front end surface. The threaded hole 28 extends in the axial direction of the operation member 23.

An operation rod 30 is inserted in the pipe holes 21a and 21b of the grip pipe 21 and the pipe hole 22a of the extension pipe 22 to be linearly movable in the axial direction of the grip pipe 21 and the extension pipe 22. The rear end of the operation rod 30 includes an external thread 31. The operation rod 30 is threaded in the threaded hole 28 of the operation member 23. The external thread 31 may be a right-hand thread or a left-hand thread. The threaded hole 28 is a right-hand thread or a left-hand thread corresponding to the thread shape of the external thread 31.

As shown in Figs. 1A and 2, the front end portion of the grip pipe 21 includes a restriction hole 33 extending in the radial direction and through the grip pipe 21. The restriction hole 33 is a slot elongated in the axial direction of the grip pipe 21.

A pin 34 extends in the radial direction from a section of the operation rod 30 facing the restriction hole 33. The pin 34 is inserted in the restriction hole 33. The pin 34 is allowed to reciprocate in the axial directions of the grip pipe 21 within the range of the length of the restriction hole 33. This allows the operation rod 30 to reciprocate in the axial directions of the grip pipe 21 within the range of the length of the restriction hole 33.

The pin 34 is in contact with the inner circumferential surface of the restriction hole 33 in the directions perpendicular to the longitudinal direction of the restriction hole 33. This restricts movement of the pin 34 in these perpendicular directions. As a result, the operation rod 30 cannot rotate about the axis of the grip pipe 21. That is, when the operation member 23 is rotated about the axis of the grip pipe 21, the operation rod 30 does not rotate together.

As such, when the operation member 23 is rotated about the axis of the grip pipe 21, the kinematic pair of the external thread 31 and the threaded hole 28 moves the operation rod 30 in an axial direction relative to the operation member 23 within the range of the length of the restriction hole 33. When the pin 34 is in contact with the rear end surface of the restriction hole 33, the pin 34 is at position P1, which is a first position of the operation rod 30. When the pin 34 is in contact with the front end surface of the restriction hole 33, the pin 34 is at position P2, which is a second position of the operation rod 30. Positions P1 and P2 of the pin 34 are not limited to the positions at which the pin 34 is in contact with the surface defining the restriction hole 33, and may be positions spaced apart from the front end surface and the rear end surface of the restriction hole 33.

### Actuation Portion 40

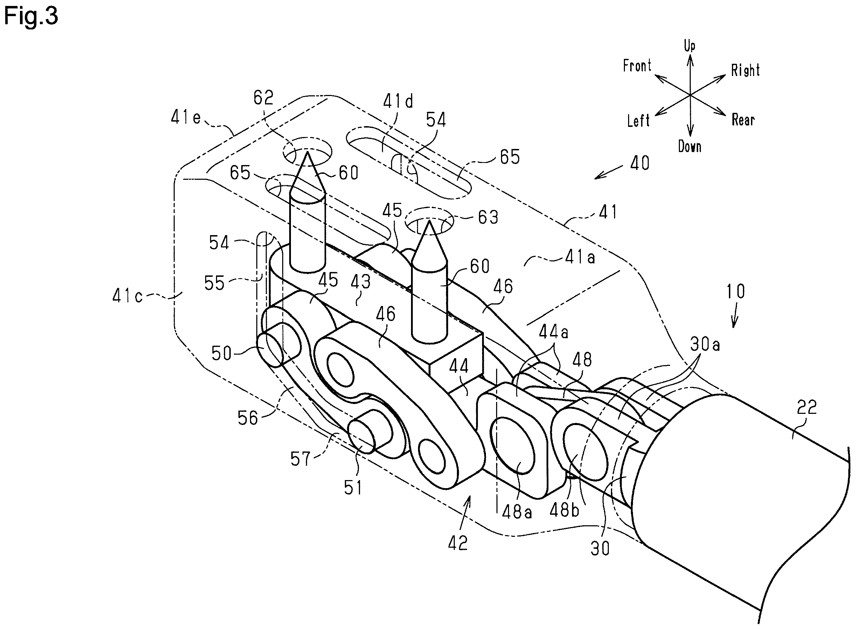

As shown in Figs. 3 to 7, the actuation portion 40 includes an actuation portion case 41, which is a case, and a mechanism portion 42 and needle members 60, which are housed in the actuation portion case 41.

As shown in Figs. 1A, 1B, and 3, the actuation portion case 41 is integrally coupled to the front end of the extension pipe 22. The actuation portion case 41 includes an upper sidewall 41a, a lower sidewall 41b, a left sidewall 41c, a right sidewall 41d, and a front wall 41e, and substantially has the shape of a rectangular solid. The rear portion of the actuation portion case 41 is tapered toward the extension pipe 22. The inside of the actuation portion case 41 communicates with the pipe hole 22a of the extension pipe 22. The vertical length (height) of the actuation portion case 41 is dimensioned to allow the actuation portion case 41 to be inserted between vertebrae.

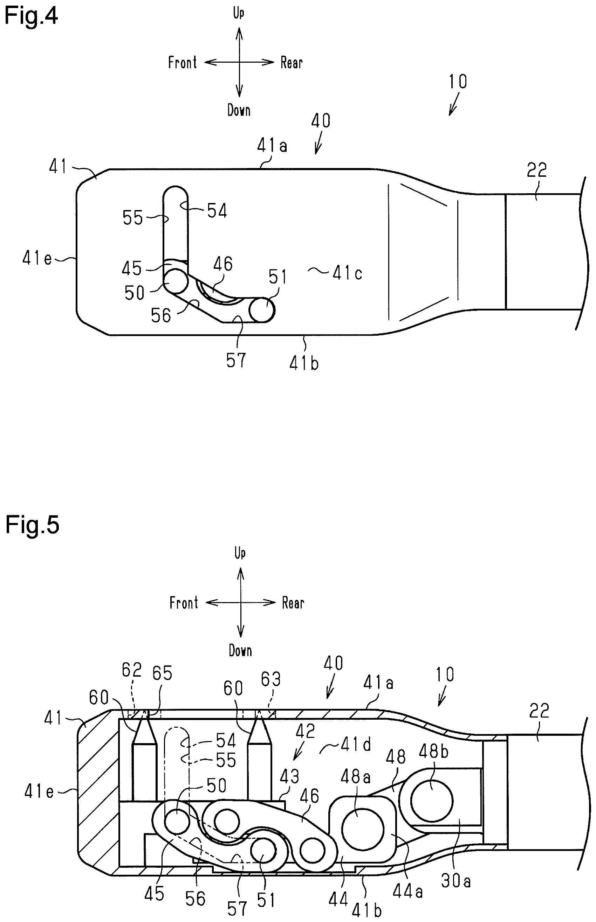

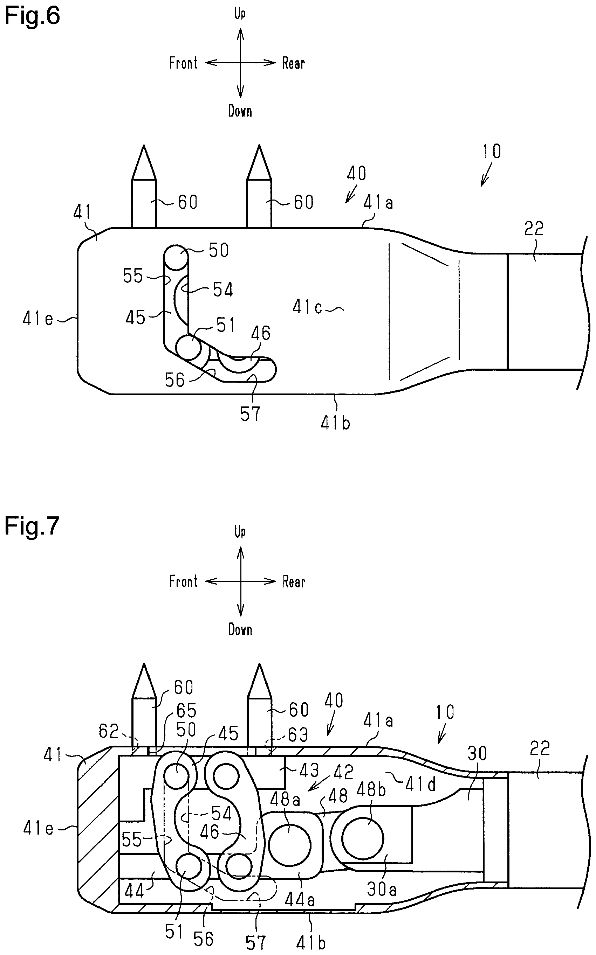

The mechanism portion 42 includes a parallel link mechanism. The mechanism portion 42 includes an upper link 43 and a lower link 44, which are parallel to each other, two front links 45, and two rear links 46. The lower link 44 is disposed under the upper link 43. The two front links 45 are disposed on the left and right sides of the upper link 43 and the lower link 44. The front links 45 are rotationally coupled to the opposite side surfaces of the upper link 43 and rotationally coupled to the opposite side surfaces of the lower link 44. The two rear links 46 are disposed on the left and right sides of the upper and lower links 43 and 44. The rear links 46 are rotationally coupled to the opposite side surfaces of the upper link 43 and rotationally coupled to the opposite side surfaces of the lower link 44. Figs. 5 and 7 show only the combination of the front and rear links 45 and 46 on the left side of the upper and lower links 43 and 44. Fig. 3 shows also the combination of the front and rear links 45 and 46 on the right side of the upper and lower links 43 and 44.

The points at which the front links 45 are coupled to the upper link 43 are located forward of the points at which the rear links 46 are coupled to the upper link 43. The points at which the front links 45 are coupled to the lower link 44 are located forward of the points at which the rear links 46 are coupled to the lower link 44. The distance between the two points at which a front link 45 is coupled to the upper and lower links 43 and 44 is the same as the distance between the two points at which a rear link 46 is coupled to the upper and lower links 43 and 44. Also, the distance between the two points at which the upper link 43 is coupled to the front and rear links 45 and 46 is the same as the distance between the two points at which the lower link 44 is coupled to the front and rear links 45 and 46.

Two needle members 60 are integrally disposed on the front portion and the rear portion of the upper link 43. The needle members 60 project from the outer surface of the upper sidewall 41a. The needle members 60 have the same thickness and the same height. As shown in Figs. 3 and 5, the upper sidewall 41a of the actuation portion case 41 includes two windows 62 and 63 in sections facing the needle members 60. The windows 62 and 63 extend through the upper sidewall 41a to provide communication between the inside and outside of the actuation portion case 41. The outer surface of the upper sidewall 41a is the side surface that faces an endplate of a vertebra during surgery. When the endplate perforator 10 is placed parallel to the endplate, the outer surface of the upper sidewall 41a serves as an insertion surface parallel to the endplate. The windows 62 and 63 are sized to allow the needle members 60 to project outward as shown in Figs. 6 and 7 when the needle members 60 move upward. The needle members 60 are movable in directions substantially perpendicular to the outer surface of the upper sidewall 41a to project and retract relative to the case 41. The outer surface of the upper sidewall 41a is parallel to the moving direction of the operation rod 30. In other words, the outer surface of the upper sidewall 41a is parallel to the axial direction of the pipe (21, 22).

As shown in Fig. 3, the upper sidewall 41a includes elongated interference avoidance holes 65 at sections facing the front and rear links 45 and 46. When the front and rear links 45 and 46 move upward, the upper parts of these links 45 and 46 are inserted into the interference avoidance holes 65 so that they do not interfere with the upper sidewall 41a.

As shown in Figs. 3, 5, and 7, a coupling link 48 is rotationally coupled to the rear end of the lower link 44 and the front end of the operation rod 30. The rear end of the lower link 44 is bifurcated and includes two coupling portions 44a. The front end of the operation rod 30 is bifurcated and includes two coupling portions 30a. The front end of the coupling link 48 is inserted between the coupling portions 44a and is pivotally coupled to the lower link 44 by a shaft 48a. The rear end of the coupling link 48 is inserted between the coupling portions 30a and is pivotally coupled to the operation rod 30 by a shaft 48b. When the operation rod 30 is operated to move from the first position to the second position, the lower link 44 is pushed via the coupling link 48 and thus moved. In contrast, when the operation rod 30 is operated to move from the second position to the first position, the lower link 44 are pulled and thus moved.

As shown in Fig. 3, the mechanism portion 42 includes shafts 50 and 51, which rotationally couple the front links 45 to the upper and lower links 43 and 44. Each end of the shafts 50 and 51 is inserted in the corresponding one of a guide groove 54 formed in the left sidewall 41c and a guide groove 54 formed in the right sidewall 41d of the actuation portion case 41. The relationship between the shafts 50 and 51 and the guide grooves 54 will be detailed below. Figs. 4 to 7 show only the guide groove 54 formed in the left sidewall 41c.

As shown in Fig. 4, each guide groove 54 includes a first guide 55, a second guide 56, and a third guide 57. The first guide 55 extends vertically. The shaft 50 is vertically movable between the upper end and the lower end of the first guide 55.

The second guide 56 is continuous with the lower end of the first guide 55 and extends obliquely downward toward the rear. That is, the front end of the second guide 56 is located above the rear end of the second guide 56. The third guide 57 is continuous with the rear end of the second guide 56 and linearly extends rearward. The shaft 51 is movable between the front end of the second guide 56 and the rear end of the third guide 57.

### Operation of Parallel Link Mechanism

Operation of the parallel link mechanism of the present embodiment is now described.

The following description mainly focuses on the movements and positions of the shafts 50 and 51 of the front links 45. The parallel link mechanism includes the upper link 43, the lower link 44, the front links 45, and the rear links 46. Thus, the description of the shafts of the rear links 46 corresponding to the shafts 50 and 51 is omitted. It should be understood that the shafts of the rear links 46 are in synchronization with the shafts 50 and 51 and are at the same heights.

As shown in Figs. 3 to 5, when the pin 34 is at position P1 shown in Fig. 2, that is, when the operation rod 30 is at the first position, the shaft 51 is at the rear end of the third guide 57, and the shaft 50 is at the lower end of the first guide 55.

When the shaft 50 is at the lower end of the first guide 55, the upper link 43 is at a lower position as shown in Figs. 3 to 5. Thus, the needle members 60 are at a retracted position, at which the needle members 60 do not project outward from the windows 62 and 63.

When the pin 34 is at position P2 shown in Fig. 2, that is, when the operation rod 30 is at the second position, the shaft 51 is positioned at the front end of the second guide 56 and the shaft 50 is at the upper end of the first guide 55 as shown in Figs. 6 and 7. Thus, the upper link 43 is positioned at an upper position as shown in Figs. 6 and 7. As a result, the needle members 60 are at a projecting position at which the needle members 60 project outward from the windows 62 and 63. This projecting position is set so that the projection amount of the needle members 60 from the upper sidewall 41a is greater than or equal to the thickness of an endplate S, which will be described below.

With the above configuration, when the operation rod 30 is operated to move the pin 34 from position P1 to position P2 shown in Fig. 2, that is, when the operation rod 30 is operated to move from the first position to the second position, the operation rod 30 presses the lower link 44 via the coupling link 48. As a result, the shaft 51 moves from the rear end to the front end of the third guide 57 and then from the rear end to the front end of the second guide 56. As the shaft 51 moves along the second guide 56, it gradually ascends and moves to the front end of the second guide 56. The forward movement and ascent of the shaft 51 move the lower link 44 forward and upward.

At the same time, the shaft 50 moves from the lower end to the upper end of the first guide 55, moving the upper link 43 to the upper position (see Fig. 7). The movement of the upper link 43 to the upper position moves the needle members 60 to the projecting position at which the needle members 60 project from the windows 62 and 63. The mechanism portion 42 converts the movement direction of the operation rod 30 so that the needle members 60 move between the retracted position and the projecting position in directions perpendicular to the axial direction of the grip pipe 21.

When the operation rod 30 is operated to move the pin 34 from position P2 to position P1 shown in Fig. 2, that is, when the operation rod 30 is operated to move from the second position to the first position, the operation rod 30 pulls the lower link 44 via the coupling link 48.

As a result, the shaft 51 moves from the front end to the rear end of the second guide 56 and then from the front end to the rear end of the third guide 57. As the shaft 51 moves along the second guide 56, it gradually descends and moves toward the rear end of the second guide 56. The rearward movement and descent of the shaft 51 move the lower link 44 rearward and downward. At the same time, the shaft 50 moves from the upper end to the lower end of the first guide 55, moving the upper link 43 to the lower position (see Fig. 5). The movement of the upper link 43 to the lower position moves the needle members 60 to the retracted position at which the needle members 60 do not project from the windows 62 and 63. The upper link 43 is engaged with the actuation portion case 41 via the shaft 50 such that movement of the upper link 43 in a direction other than the directions perpendicular to the outer surface of the upper sidewall 41a, which is the insertion surface, is restricted.

### Operation of First Embodiment

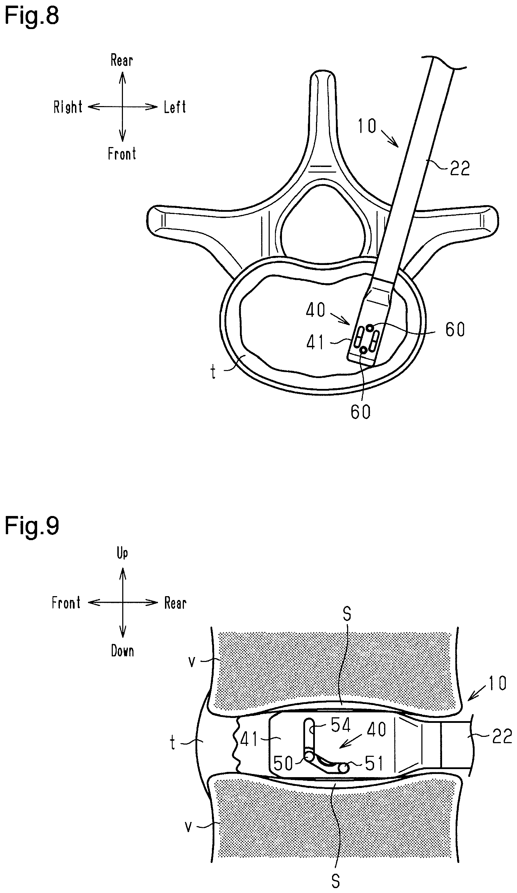

Referring to Figs. 8 to 12, a method for using the endplate perforator 10 configured as described above is now described.

As shown in Figs. 8 and 9, the surgeon inserts the endplate perforator 10, with the needle members 60 in the retracted position facing upward, into a space formed by dissecting the intervertebral tissue t with a punch (not shown) from the patient's dorsal side. Then, as shown in Fig. 9, the surgeon positions the needle members 60 at the retracted position of the endplate perforator 10 so that they face the endplate S of the upper vertebral body v. In this state, the surgeon rotates the operation handle 24 and the operation member 23 shown in Fig. 1 to move the operation rod 30 from the first position to the second position.

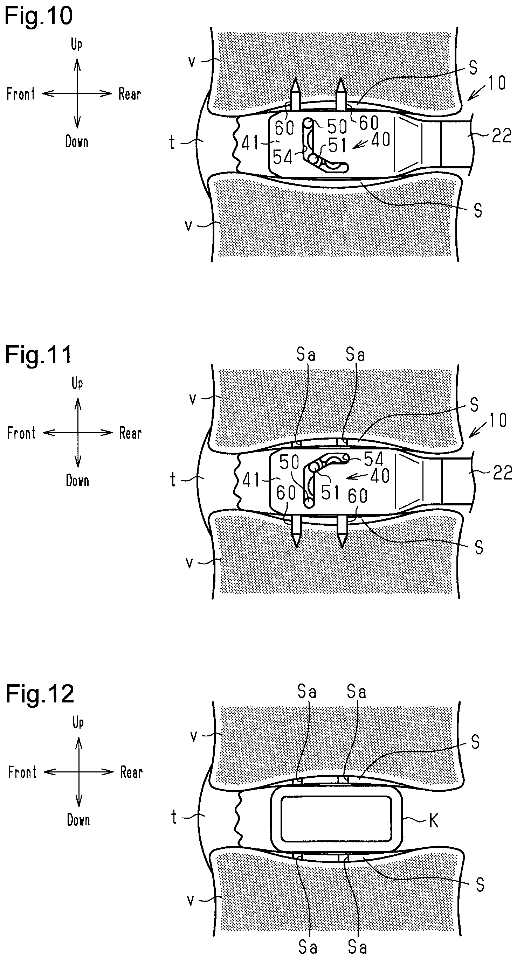

As a result, the operation rod 30 presses the lower link 44 of the parallel link mechanism via the coupling link 48, thereby moving the upper link 43 upward and positioning the needle members 60 at the projecting position. As shown in Fig. 10, the needle members 60 thus penetrate the endplate S of the upper vertebral body v and form bone tunnels Sa substantially perpendicular to the endplate S.

Subsequently, the surgeon rotates the operation handle 24 and the operation member 23 shown in Fig. 1 in the direction opposite to the operation that causes the needle members 60 to project as described above, thereby moving the operation rod 30 from the second position to the first position. As a result, the operation rod 30 pulls the lower link 44 of the parallel link mechanism via the coupling link 48, thereby moving the upper link 43 downward and positioning the needle members 60 at the retracted position.

The surgeon then removes the endplate perforator 10 from the patient, turns the endplate perforator 10 upside down by 180 degrees to change its orientation to downward orientation. The endplate perforator 10 is inserted again from the dorsal side so that the needle members 60 at the retracted position face the endplate S of the lower vertebral body v.

In this state, the surgeon rotates the operation handle 24 and the operation member 23 shown in Fig. 1 to move the operation rod 30 from the first position to the second position. As a result, the operation rod 30 presses the lower link 44 of the parallel link mechanism via the coupling link 48, and the needle members 60 moves from the retracted position to the projecting position. As shown in Fig. 11, the needle members 60 penetrate the endplate S of the lower vertebral body v and form bone tunnels Sa substantially perpendicular to the endplate S.

Subsequently, the surgeon rotates the operation handle 24 shown in Fig. 1 in the direction opposite to the operation that causes the needle members 60 to project as described above, thereby moving the operation rod 30 from the second position to the first position. As a result, the operation rod 30 pulls the lower link 44 of the parallel link mechanism via the coupling link 48, thereby positioning the needle members 60 at the retracted position. Then, as shown in Fig. 12, the surgeon removes the endplate perforator 10 from between the vertebral bodies, inserts a cage K into the space between the vertebral bodies using a cage holder (not shown), and implants bone grafts in the cage K and between vertebral bodies. Since the bone tunnels Sa are formed substantially perpendicularly to the endplates S as described above, bone marrow blood in the cancellous tissue of the vertebral bodies v is introduced into the bone grafts between the vertebral bodies through these bone tunnels Sa. This facilitates bone fusion between the vertebral bodies, thereby fusing the vertebrae.

The present embodiment includes the following features.
(1) The endplate perforator 10 includes the actuation portion case 41, which includes the upper sidewall 41a including an outer surface that faces an endplate of a vertebra when the endplate perforator 10 is inserted between adjacent vertebrae, the grip pipe 21 and the extension pipe 22, which form a pipe integral with the actuation portion case 41, and the operation rod 30, which is inserted in the grip pipe 21 and the extension pipe 22 to be reciprocally movable. The endplate perforator 10 also includes the needle members 60, which are movable between the retracted position, at which the needle members 60 are retracted in the actuation portion case 41, and the projecting position, at which the needle members 60 project from the upper sidewall 41a. The endplate perforator 10 includes the mechanism portion 42, which is disposed in the actuation portion case 41 and coupled to the needle members 60 and the operation rod 30 in an operable manner. The mechanism portion 42 is configured to reciprocate the needle members 60 between the retracted position and the projecting position by reciprocating movement of the operation rod 30.
   According to the above configuration, when the operation rod 30 is operated to move in one direction after the actuation portion case 41 is inserted between vertebrae and the upper sidewall 41a of the actuation portion case 41 is positioned to face an endplate of a vertebra, the mechanism portion 42 moves the needle members 60 from the retracted position to the projecting position. When moved to the projecting position, the needle members 60 perforate the endplate of the vertebra facing the outer surface of the upper sidewall 41a of the actuation portion case 41 while being substantially perpendicular to the endplate. When the operation rod 30 is operated to move in the direction opposite to the above direction after the endplate is perforated, the mechanism portion 42 moves the needle members 60 from the projecting position to the retracted position. The endplate perforator 10 used as described above in a posterior interbody fusion suitably and easily forms bone tunnels that are substantially perpendicular to the endplate. These bone tunnels facilitate the bone fusion between the vertebrae.
(2) The operation rod 30 is linearly movable in the axial directions of the grip pipe 21 and the extension pipe 22. Also, the needle members 60 are movable between the projecting position and the retracted position by reciprocating in directions perpendicular to the moving directions of the operation rod 30. The mechanism portion 42 converts linear movement of the operation rod 30 into movement of the needle members 60 in a direction perpendicular to the moving direction of the operation rod 30.
   According to the above configuration, the needle members 60 project and perforate an endplate of a vertebra with the outer surface of the actuation portion case 41 of the endplate perforator 10 positioned substantially parallel to the endplate of the vertebra.
(3) The mechanism portion 42 is a parallel link mechanism. This mechanism facilitates the achievement of advantage (2) above.
(4) The front end of the operation member 23 is inserted in the grip pipe 21 to be rotatable about the axis of the grip pipe 21. One end of the operation rod 30 is threaded into the front end of the operation member 23. Thus, by rotating the operation member 23 about the axis of the grip pipe 21, the operation rod 30 reciprocates between the first position and the second position.
   According to the above configuration, the mechanism portion 42 allows the needle members 60 to reciprocate between the retracted position and the projecting position.

### Second Embodiment

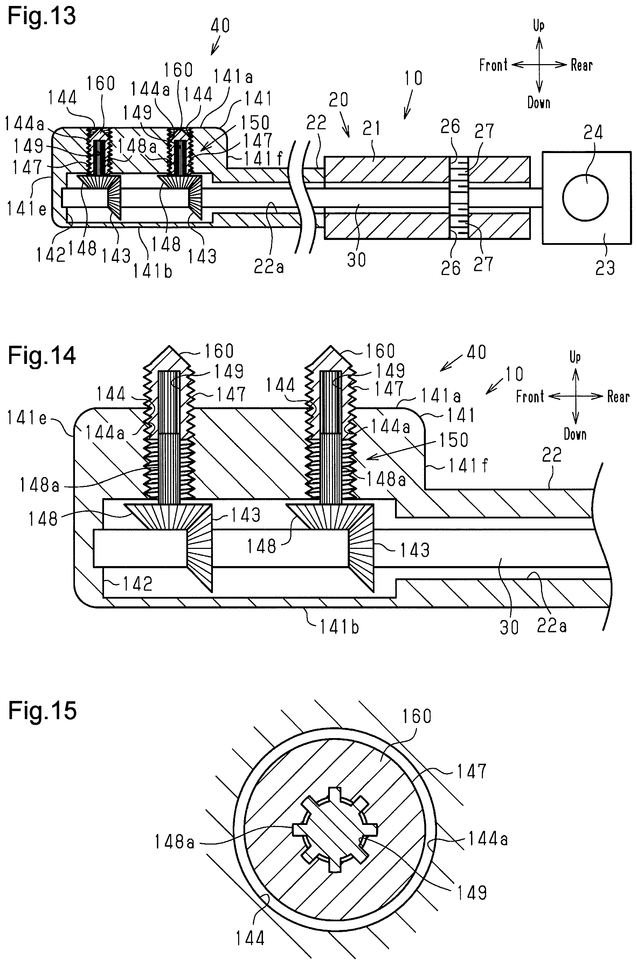

Referring to Figs. 13 to 15, an endplate perforator 10 according to a second embodiment is now described. Same reference numerals are given to those configurations that are the same as or correspond to those in the first embodiment. Such configurations will not be described in detail.

Referring to Fig. 13, the present embodiment differs from the endplate perforator 10 of the first embodiment in the following points. The rear end of the operation rod 30 projects from the grip pipe 21. An operation member 23, which is shorter than that of the first embodiment, is integrally fixed to the rear end of the operation rod 30. The operation rod 30 includes a circumferential groove. This circumferential groove is formed in a section of the outer circumferential surface of the operation rod 30 that is located within the grip pipe 21. Two threaded holes 26 extend through the grip pipe 21 in the radial direction. A setscrew 27 is threaded in each threaded hole 26. The distal end of the setscrew 27 is inserted in the circumferential groove of the operation rod 30. Thus, the operation rod 30 is prevented from moving in the axial directions of the grip pipe 21 while being allowed to rotate about the axis of the grip pipe 21.

The actuation portion 40 at the front end of the extension pipe 22 includes an actuation portion case 141, which is a case, and a mechanism portion 150 and needle members 160, which are housed in the actuation portion case 141.

The actuation portion case 141 is formed by a rectangular block and includes an upper side surface 141a, a lower side surface 141b, a left side surface, a right side surface, a front side surface 141e, and a rear side surface 141f. The actuation portion case 141 includes a bevel gear housing hole 142 extending in the front-rear direction. The bevel gear housing hole 142 houses two front and rear driving bevel gears 143, which are integrally coupled to the front end of the operation rod 30.

The actuation portion case 141 also includes two needle member housing holes 144 communicating with the bevel gear housing hole 142. The needle member housing holes 144 open at the upper side surface 141a and are arranged in the front-rear direction. The upper side surface 141a is the side surface facing an endplate during surgery. The upper side surface 141a serves as an insertion surface that is placed parallel to the endplate during surgery.

Each needle member housing hole 144 houses a needle member 160, which moves up and down while rotating. Thus, the needle members 160 are movable in directions substantially perpendicular to the upper side surface 141a to project and retract relative to the actuation portion case 141. That is, the inner surface of each needle member housing hole 144 includes a first spiral groove 144a, and the outer circumferential surface of the section of each needle member 160 other than the tip portion includes a second spiral groove 147. The needle member 160 is threaded in the needle member housing hole 144. The needle member 160 includes a spline shaft housing hole 149 opening at its proximal end surface. The spline shaft housing hole 149 extends in an axial direction of the needle member 160.

The mechanism portion 150 includes the driving bevel gears 143, driven bevel gears 148 meshing with the driving bevel gears 143, and spline shafts 148a integral with the driven bevel gears 148. As shown in Fig. 15, the outer circumferential surface of each spline shaft 148a includes spline teeth protruding radially outward, and the inner circumferential surface of each spline shaft housing hole 149 includes spline teeth protruding radially inward. The spline shaft 148a is fitted in the spline shaft housing hole 149, thereby allowing the needle member 160 to move relative to the spline shaft 148a in the axial directions. The driving bevel gears 143 and the driven bevel gears 148 constitute a bevel gear mechanism.

When the operation handle 24 and the operation member 23 are rotated to rotate the driving bevel gears 143 via the operation rod 30, the rotational movement is transmitted to the driven bevel gears 148. The spline shafts 148a on the driven bevel gears 148 then transmit the rotational movement to the needle members 160, thereby rotating the needle members 160. When the needle members 160 are rotated, the vertical driving forces provided by the spiral grooves 144a and 147 cause the needle members 160 to move, while rotating, from the retracted position, at which the needle members 160 are retracted in the actuation portion case 141 as shown in Fig. 13, to the projecting position, at which the needle members 160 project outward from the actuation portion case 141 as shown in Fig. 14.

With the endplate perforator 10 configured as described above, in the same manner as the first embodiment, moving the needle members 160 from the retracted position to the projecting position with the needle members 160 facing upward allows the needle members 160 to penetrate through an endplate S of the upper vertebral body v, thereby forming bone tunnels substantially perpendicular to the endplate S. Likewise, moving the needle members 160 from the retracted position to the projecting position with the needle members 160 facing downward allows the needle members 160 to penetrate an endplate S of the lower vertebral body v, thereby forming bone tunnels substantially perpendicular to the endplate S.

The endplate perforator 10 of the present embodiment allows the needle members 160 to move while rotating, so that bone tunnels are reliably formed in the endplates of the vertebrae. The needle members 160 may be drill-shaped.

### Third Embodiment

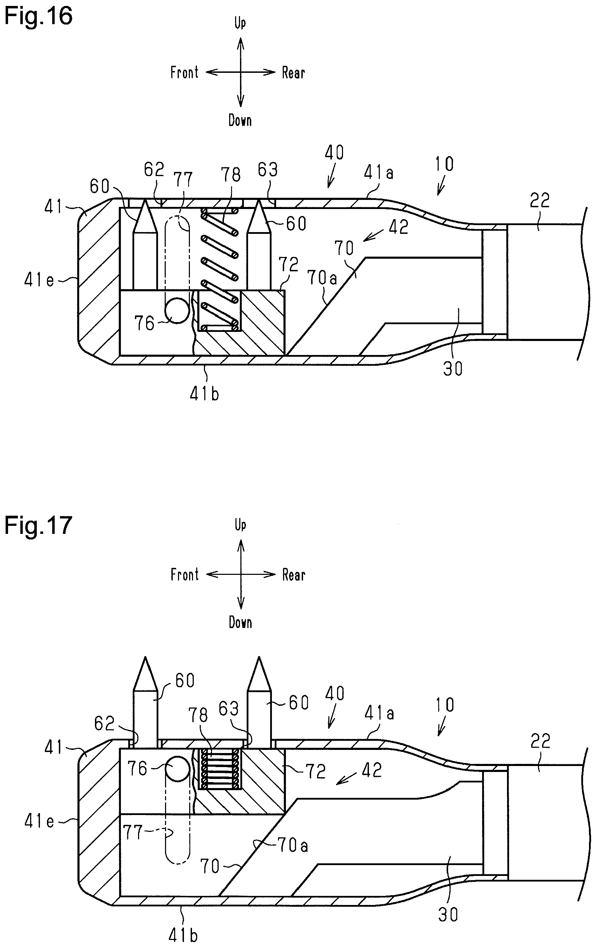

Referring to Figs. 16 and 17, a third embodiment is now described. The present embodiment differs from the endplate perforator 10 of the first embodiment in that a cam mechanism is employed as the mechanism portion 42. The cam mechanism may be a linear cam, a front cam, or a plate cam, for example.

Fig. 16 shows a linear cam as an example of the cam mechanism. The cam mechanism includes a cam plate 70, which is integral with the front end of the operation rod 30, a block-shaped cam follower 72, and a coil spring 78, which is an urging member. The front surface of the cam plate 70 serves as a cam surface 70a. The cam surface 70a is flat and inclined so that the height decreases toward the front.

The two needle members 60 are arranged in the front-rear direction on the upper surface of the cam follower 72. The lower edge of the rear end of the cam follower 72 is in contact with the cam surface 70a of the cam plate 70. When the operation rod 30 moves frontward, the cam follower 72 slides on the cam surface 70a and thus moves upward. The front surface of the cam follower 72 is in contact with the flat inner surface of the front wall 41e to be vertically slidable. That is, the cam follower 72 is sandwiched in the front-rear direction between the inner surface of the front wall 41e and the cam surface 70a. Since the cam follower 72 is always in contact with the cam surface 70a and the inner surface of the front wall 41e, the needle members 60 are equal in height. Two guide protrusions 76 extend to the left and right from the left and right side surfaces of the cam follower 72. Each of the left and right sidewalls of the actuation portion case 41 includes a guide hole 77. The guide hole 77 extends in the vertical direction. The guide protrusions 76 are slidably inserted in the guide holes 77. The cam follower 72 engages with the actuation portion case 41 via the guide protrusions 76 such that movement of the cam follower 72 in a direction other than the directions perpendicular to the outer surface of the upper sidewall 41a, which is the insertion surface, is restricted.

As shown in Fig. 16, when the operation rod 30 is at the first position as in the first embodiment (Fig. 2), the needle members 60 are at the retracted position. Also, as shown in Fig. 17, when the operation rod 30 is at the second position as in the first embodiment, the needle members 60 are at the projecting position projecting from the upper sidewall 41a.

The coil spring 78 is placed between the upper surface of the cam follower 72 and the inner surface of the upper sidewall 41a. The coil spring 78 always urges the cam follower 72 downward. That is, the coil spring 78 urges the cam follower 72 in the direction opposite to the direction in which the needle members 60 project. The above configuration has advantage (1) of the first embodiment.

### Results of Actual Usage Example

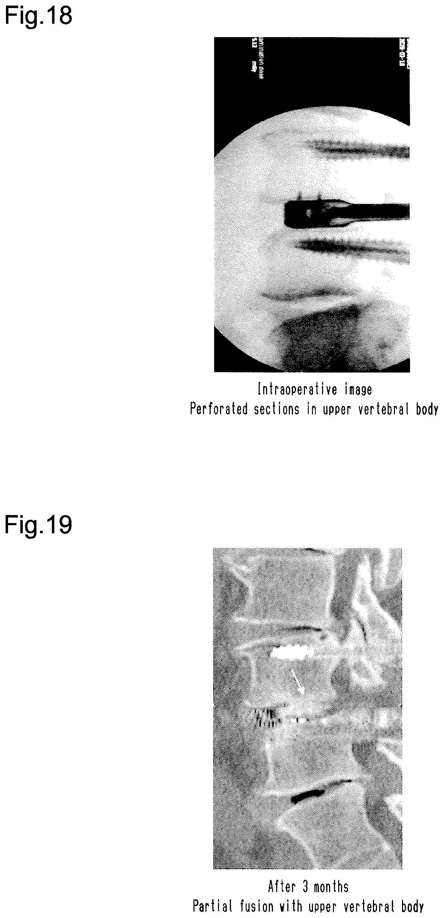

Fig. 18 is an intraoperative image in which the endplate perforator of the first embodiment perforated the upper vertebral body, thereby making holes in the endplate. As shown in Fig. 18, the holes were formed substantially perpendicularly to the endplate. Three months after performing bone grafting between the vertebrae in this surgery, bone fusion was observed between a vertebral body and a part of the bone between the vertebrae as indicated by the arrow in Fig. 19.

The above result indicates that bone marrow blood in the cancellous tissue of the vertebral bodies was introduced into the bone graft between the vertebral bodies through the holes formed in the endplates by the endplate perforator, allowing the bone fusion to occur earlier than the conventional example described in the BACKGROUND ART section.

The above-described embodiments may be modified and implemented as follows.

The embodiments and the following modifications may be combined to the extent that does not cause technical contradiction.

For example, the configuration of the first embodiment may further include a second parallel link mechanism actuated by the operation rod 30, which reciprocates linearly, and a second needle member, which is moved by the second parallel link mechanism to project downward from the lower sidewall 41b.

The following technical concepts may be derived from the embodiments and modifications described above.
[Additional Statement 1] The first link may be engaged with the case such that movement of the first link in a direction other than the directions perpendicular to the insertion surface is restricted.
[Additional Statement 2] The cam follower may be engaged with the case such that movement of the cam follower in a direction other than the directions perpendicular to the insertion surface is restricted.
[Additional Statement 3] An interbody fusion using an endplate perforator, wherein
   the endplate perforator includes:
      a pipe,
      an operation portion disposed at a rear end of the pipe;
      a case that is disposed at a front end of the pipe and includes an insertion surface;
      a needle member housed in the case and configured to perforate an endplate of a vertebra; and
      a mechanism portion configured to move the needle member in a direction perpendicular to the insertion surface in response to an operation of the operation portion to cause the needle member to project or retract relative to the case, and
   the interbody fusion may include:
      inserting the case into a space formed by dissecting intervertebral tissue such that the insertion surface is parallel to the endplate of the vertebra; and
      perforating the endplate by projecting the needle member in a direction perpendicular to the insertion surface.
[Additional Statement 4] The projecting the needle member in a direction perpendicular to the insertion surface may include rotating the operation portion.

## Claims

1. An endplate perforator comprising:
a pipe,
an operation portion disposed at a rear end of the pipe;
a case disposed at a front end of the pipe, the case including an insertion surface configured to be placed parallel to an endplate of a vertebra;
a needle member housed in the case and configured to perforate the endplate of the vertebra; and
a mechanism portion configured to move the needle member in a direction perpendicular to the insertion surface in response to an operation of the operation portion to cause the needle member to project or retract relative to the case.

2. The endplate perforator according to claim 1, wherein
the operation portion includes:
an operation member configured to rotate relative to the pipe about an axis of the pipe; and
an operation rod configured to move linearly in an axial direction of the pipe in response to rotation of the operation member, and
the mechanism portion is configured to convert linear movement of the operation rod into movement of the needle member in a direction perpendicular to the insertion plane.

3. The endplate perforator according to claim 2, wherein the mechanism portion includes a parallel link mechanism including:
a first link on which the needle member is disposed;
a second link parallel to the first link and coupled to the operation rod;
a third link rotationally coupled to the first link and the second link; and
a fourth link rotationally coupled to the first link and the second link.

4. The endplate perforator according to claim 2, wherein the mechanism portion includes a linear cam including:
a cam plate that is coupled to the operation rod and includes a cam surface inclined with respect to a moving direction of the operation rod;
a cam follower on which the needle member is disposed, the cam follower being in contact with the cam surface; and
an urging member that urges the cam follower in a direction in which the needle member retracts into the case.

5. The endplate perforator according to claim 1, wherein
the operation portion includes:
an operation member configured to rotate relative to the pipe about an axis of the pipe; and
an operation rod coupled to the operation member to rotate integrally with the operation member,
the case includes a needle member housing hole that opens in the insertion surface and includes an inner surface including a first spiral groove,
the needle member includes:
a second spiral groove that meshes with the first spiral groove; and
a spline shaft housing hole that opens in a proximal end surface of the needle member and extends in an axial direction of the needle member, and the mechanism portion includes a bevel gear mechanism including:
a driving bevel gear coupled to the operation rod to rotate integrally with the operation rod;
a driven bevel gear meshing with the driving bevel gear; and
a spline shaft that is coupled to the driven bevel gear to rotate integrally with the driven bevel gear and is fitted in the spline shaft housing hole.
